# EUROPEAN PATENT APPLICATION

(11) **EP 4 379 041 A1**
(43) Date of publication of application: **05.06.2024**
(21) Application number: 22211187.4
(22) Date of filing: 02.12.2022
(51) Int. Cl.: C12M 1/42, C12M 1/12

(54) **MECHANICAL STRETCHING SYSTEM FOR MIMICKING PHYSIOLOGICAL ENVIRONMENT**

(71) Applicant: Fondazione Istituto Firc Di Oncologia Molecolare (IFOM), 20139 Milano (IT)
(72) Inventor: LI, Qingsen, 20139 Milano (MI) (IT)
(74) Representative: Ghirardi, Valeria

(57) **Abstract**

Cell stretching device (1) comprising a housing (2) having walls (3) defining a hollow region (4), a first deformable membrane (5) and a second deformable membrane (6) facing the first deformable membrane (5), both the first deformable membrane (5) and the second deformable membrane (6) being located in the housing (2) and being fixable to the walls (3) of the housing (2), and an air chamber (8) formed in an internal region of the housing (2) and defined by the first deformable membrane (5), the second deformable membrane (6), and a central portion (9) of the walls (3) of the housing (2), said central portion (9) of the walls (3) comprising at least a vacuum port (10) for evacuating air from the air chamber (8) and to determine a pressure variation inside the air chamber (8), wherein in a first configuration the air pressure inside the air chamber (8) is such that the first deformable membrane (5) is separated from the second deformable membrane (6) at a predefined membrane distance (d), and in a second configuration the air pressure inside the air chamber (8) is reduced such that the first deformable membrane (5) and the second deformable membrane (6) are deformed and stretched towards the central portion (9) of the walls (3) of the housing (2) and such that the first deformable membrane (5) is, at least partially, in contact with the second deformable membrane (6) to form a stretching region.

## Description

The invention relates to a cell stretching device and to a cell stretching plate comprising a plurality of said cell stretching devices. Also, the invention relates to a method for cell stretching using said device or said plate and to a process for retrofitting a cell stretching system using a vacuum unit.

Cells in the body constantly experience mechanical stimuli in various organ movement, development and functions. These mechanical stimuli are often essential for several cell functions, such as migration, proliferation, differentiation. Cells sense such mechanical stimuli through a process called mechanotransduction. Abnormal mechanotransduction may lead to several pathologies, such as cancer, asthma, and heart disease. In mechanobiology studies, the advance of *in vitro* technologies for the application of physiologically mimicking mechanical force to cultured cells and tissue are vital to mimic the complex *in vivo* biological system. The growing interest in mechanobiology and technology gap have motivated the development of cell stretching devices in various research labs and companies.

In particular, US 6,218,178 and US 6,645,759 disclose both cell stretching systems that use a vacuum to stretch the membrane. However, these type of cell stretching systems require a loading post to support and maintain the membrane flat in the stretching region.

This may lead to some disadvantages. First of all, the loading post underneath the membrane can limit the maximum stretching level and can make the system unsuitable for imaging. Also, the friction between the loading post and the membrane requires lubricant, which affects the stretching precision. In addition, the system cannot be miniaturized for high throughput screening due to loading post and friction.

Examples of the present disclosure seek to address or at least alleviate the above problems.

According to a first aspect of the invention there is provided a cell stretching device comprising:
a housing having walls defining a hollow region;
a first deformable membrane and a second deformable membrane facing the first deformable membrane, both the first deformable membrane and the second deformable membrane being located in the housing and being fixable to the walls of the housing; and
an air chamber formed in an internal region of the housing and defined by the first deformable membrane, the second deformable membrane, and a central portion of the walls of the housing, said central portion of the walls comprising at least a vacuum port for evacuating air from the air chamber and to determine a pressure variation inside the air chamber,
wherein in a first configuration the air pressure inside the air chamber is such that the first deformable membrane is separated from the second deformable membrane at a predefined membrane distance, and in a second configuration the air pressure inside the air chamber is reduced such that the first deformable membrane and the second deformable membrane are deformed and stretched towards the central portion of the walls of the housing and such that the first deformable membrane is, at least partially, in contact with the second deformable membrane to form a stretching region.

In a second aspect of the invention there is provided a plate for cell stretching comprising: a plurality of cell stretching devices according to the first aspect; and at least a frame configured to position each of the plurality of cell stretching devices arranged in rows and columns.

In a third aspect of the invention there is provided a method for cell stretching using the cell stretching device according to the first aspect, or the plate according to the second aspect, the method comprising:
providing at least one cell stretching device or the plate for cell stretching;
seeding one of the first or the second deformable membrane with cells to be analyzed, thereby forming a deformable cell substrate;
incubating the cells for a first predetermined period of time; and
stretching the cell substrate by cyclic aspiration and release of air through the vacuum port of the device or plate for a second period of time.

In a fourth aspect of the invention there is provided a process for retrofitting a cell stretching system that uses a vacuum unit for generating a stretching on a deformable cell substrate, said cell stretching system comprising an apparatus supporting the deformable cell substrate and being configured to be connected to said vacuum unit, the process comprising replacing the apparatus with the cell stretching device according to the first aspect or the cell stretching plate according to the second aspect by connecting the vacuum unit to the vacuum port of the cell stretching device.

Other aspects and features are defined in the appended claims.

Examples of the disclosure may make it possible to carry out a stretching of cells in an *in-vitro* context using a device functioning without loading posts. Accordingly, the device is easy to use and at the same time precise in applying a stretching force to the cells. Also, the device is suitable for imaging due to an increased maximum stretching level and can be miniaturized for high throughput screening.

Examples of the disclosure will now be described by way of example only with reference to the accompanying drawings, in which like references refer to like parts, and in which:
- Figure 1: shows a schematic representation of the cell stretching device according to an example.
- Figures 2A-B: show a schematic perspective representation of the cell stretching device according to an example in exploded view (Fig. 2A) and according to another example (Fig. 2B).
- Figures 3A-C: show a schematic representation of the plate according to two examples.
- Figure 4A-B: show a configuration characterization of the cell stretching device according to examples.
- Figure 5: shows a flow chart of a cell stretching method according to an example.
- Figure 6: shows a schematic representation of the steps of a retrofitting process according to an example.

A cell stretching device, a plate, a method for cell stretching as well as a retrofitting process are disclosed. In the following description, a number of specific details are presented in order to provide a thorough understanding of the examples of the disclosure. It will be apparent however to a person skilled in the art that these specific details need not be employed in order to practice the examples of the disclosure. Conversely, specific details known to the person skilled in the art are omitted for the purposes of clarity in presenting the examples.

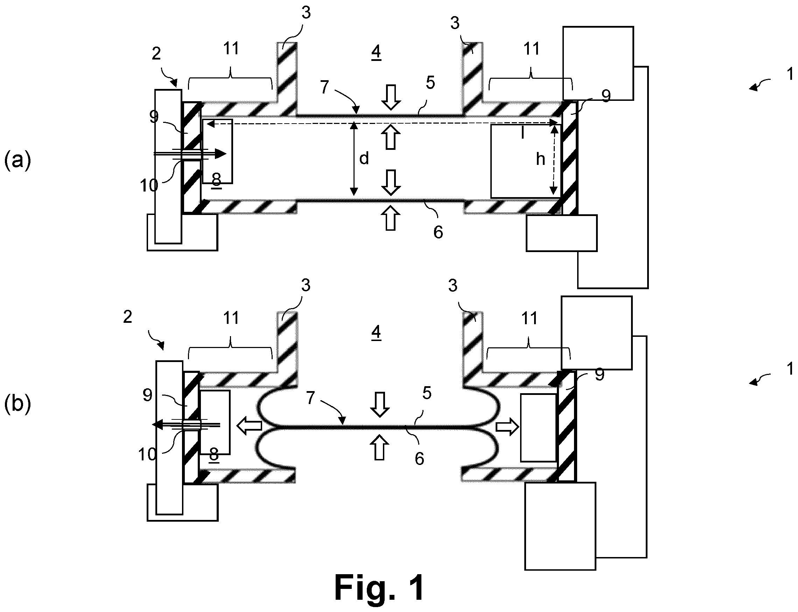

With reference to figure 1, a cell stretching device 1 is shown. The cell stretching device 1 comprises a housing 2 having basically a cylindrical shape defined by confining walls 3. In principle, the housing 2 can assume any other suitable shape providing that the housing 2 has a hollow structure and comprises a hollow region 4. The device 1 comprises two deformable membranes, a first membrane 5 and a second membrane 6 both fixed to the walls 3 of the housing 2. The first membrane 5 faces the second membrane 6 and defines, together with a central portion 9 of the walls 3 of the housing 2, an air chamber 8. In other words, the air chamber 8 is formed between the two membranes 5, 6 and the central portion 9 of the walls 3 of the housing 2 in an internal region of the housing 2. In order to evacuate air from the air chamber 8 and therefore to change, in particular reduce, the air pressure inside the chamber 8, the central portion 9 comprises at least a vacuum port 10. Accordingly, the air chamber 8 can be connected to a vacuum unit comprising a vacuum pump through the vacuum port 10. As the air chamber 10 is vacuumed, the membranes 5, 6 are deformed by atmospheric pressure and press/support each other to form a flat and stretched region.

Referring to figure 1, in a first configuration (a), the air pressure inside the air chamber 8 is such that the two membranes 5, 6 are not deformed. For example, air is not evacuated (or partially evacuated) from the air chamber 8, meaning that the air chamber 8 is at the atmospheric pressure or at a pressure value close to the atmospheric pressure such that the pressure exerted on the internal surfaces of the two membranes 5, 6 compensates (or almost compensate) the pressure exerted on the external surfaces of the two membranes 5, 6, as schematically shown by the vertical opposing arrows in the figure. The internal surface of the membranes 5, 6 indicates the side of the membranes 5, 6 facing the air chamber 8, whereas the external surface of the membranes 5, 6 indicates the side of the membranes 5, 6 opposite to the internal surface, i.e. outside the air chamber 8. The arrow shown in the vacuum port 10 pointing to the right in the figure means that in the first configuration air can be inserted into the air chamber 8 from outside in order to compensate the atmospheric pressure exerted on the external surfaces of the first and second membranes 5, 6, for example if the air was first evacuated from the chamber 8. In the first configuration (a), the first deformable membrane 5 is separated from the second deformable membrane 6 at a predefined membrane distance d. If the pressure inside the air chamber 8 equals the external pressure on the membranes 5, 6, e.g., the atmospheric pressure, the two membranes 5, 6 are flat and the membrane distance d basically corresponds to the height h of the air chamber 8.

By reducing the air pressure inside the air chamber 8, i.e. by evacuating air from the chamber 8, the two membranes 5, 6 are deformed since the external pressure is greater than the pressure inside the air chamber 8 and pushes on the external surfaces of the membranes 5, 6. Therefore, the two membranes 5, 6 are going to move closer and the membrane distance d is reduced. In particular, in a second configuration (b), air is evacuated (or totally evacuated) from the air chamber 8 such that the air chamber 8 is at a much lower pressure compared to the pressure exerted on the external surfaces of the membranes 5, 6. The air evacuation is indicated by the arrow in the vacuum port 10 pointing to the left. Accordingly, the atmospheric pressure deforms the two membranes 5, 6 so that they press or support each other, i.e. the membranes 5, 6 touch each other, and the membrane distance d is zero. In particular, the first deformable membrane 5 and the second deformable membrane 6 are deformed and stretched towards the central portion 9 of the walls 3 of the housing 2. Here, the first deformable membrane 5 is, at least partially, in contact with the second deformable membrane 6 to form a stretching region. The two opposite horizontal small arrows at the membranes 5, 6 indicate the stretching action due to the vacuumed air chamber 8, whereas the two opposite vertical small arrows at the membranes 5, 6 indicate the external pressure exerted on the membrane surfaces.

In examples, the first deformable membrane 5 or the second deformable membrane 6 is a deformable cell substrate 7 for growing cultured cells and a portion of the hollow region 4 represents a cell seeding chamber. With reference to figure 1, the first deformable membrane 5 (or upper membrane) is used as a deformable cell substrate 7, meaning that cells to be analyzed are seeded and growth on the external surface of the first membrane 5. Accordingly, the hollow region 4 defined by the first deformable membrane 5 and the lateral walls 3 of the housing 2 represents a cell seeding chamber.

The air chamber 8 has an average length I and an average height h. For example, the air chamber 8 can have a cylindrical shape so that the average length I represents the diameter of the cylinder base and the average height h represents the height of said cylinder.

In examples, the height h of the air chamber 8 is the predefined membrane distance d between the first deformable membrane 5 and the second deformable membrane 6 in the first configuration. As a matter of fact, if the pressure inside the air chamber 8 is equal or almost equal to the external pressure, the membranes 5, 6 are flat and their distance corresponds to the height of the air chamber 8, for example the cylinder height.

The ratio between the height h and the length I of the air chamber 8 can assume any reasonable value. However, in examples, the ratio between the height h and the length I of the air chamber 8 is comprised between 4:40 (i.e. 1/10) and 8:40 (i.e. 1/5), in particular 6:40 (i.e. 3/20).

In examples, the maximum stretching level of the cell stretching device 1 increases as a function of at least one of the length I of the air chamber 8, the membrane distance d, and the membrane thickness, at a fixed vacuum value inside the air chamber 8. The maximum stretching level is defined as the maximum possible stretching effect achieved on the membrane as a consequence of maximum pressure reduction inside the air chamber 8.

As a matter of fact, the maximum stretching level increases by increasing the value of the membrane distance d, the membrane thickness being the same. The maximum stretching level increases by reducing the membrane thickness, the value of the membrane distance d being the same.

In examples, the thickness of the first deformable membrane 5 and/or of the second deformable membrane 6 is comprised between 0.1 µm and 2000 µm, preferably between 1 µm and 1000 µm, in particular 100 µm or 200 µm. Specifically, it is noted that an optimal maximum stretching level is achieved with a membrane thickness of 100 µm or close to 100 µm.

In examples, the predefined membrane distance d in the first configuration is comprised between 0.1 mm and 200 mm, preferably 1 mm to 100 mm in particular 4 mm or 6 mm. Specifically, it is noted that an optimal maximum stretching level is achieved with a membrane distance d of 4 mm or close to 4 mm.

In examples, the first deformable membrane 5 and/or the second deformable membrane 6 has a diameter comprised between 1 mm and 100 mm, in particular 40 mm or 2mm, 4mm, 8mm, 16mm, 35mm.

In examples, the housing 2 comprises a protruded region 11 for receiving a portion of the first deformable membrane 5 and of the second deformable membrane 6 when stretched in the second configuration. As shown in figure 1, the protruded region 11 is defined by the central portion 9 of the walls 3 of the housing 2 that confine the air chamber 8. In particular, the protruded region 11 represents an enlarged part or a recess of the housing 2 to house a part of the deformable membranes 5, 6 when stretched. For example, if the housing 2 has a cylindrical shape, the air chamber 8 and the hollow region/cell seeding chamber 4 have both a cylindrical hollow structure, whereas the cylindrical structure defining the air chamber 8 has a larger base compared to the cylindrical structure defining the hollow region/cell seeding chamber 4. The difference in dimension of the base between these two cylindrical structures represents the extension of the protruded region 11.

In examples, the stretching region is a flat region and in the second configuration the first and the second deformable membranes 5, 6 are exclusively in contact with each other and with the walls 3 of the housing 2. In other words, the flatness of the stretching region and therefore of the cell substrate 7 facilitates the adhesion of the cells on the membrane 5, 6. Also, the particular configuration, wherein the two membranes are only in contact with each other (i.e. one membrane support the other membrane), eliminates the necessity of a loading post.

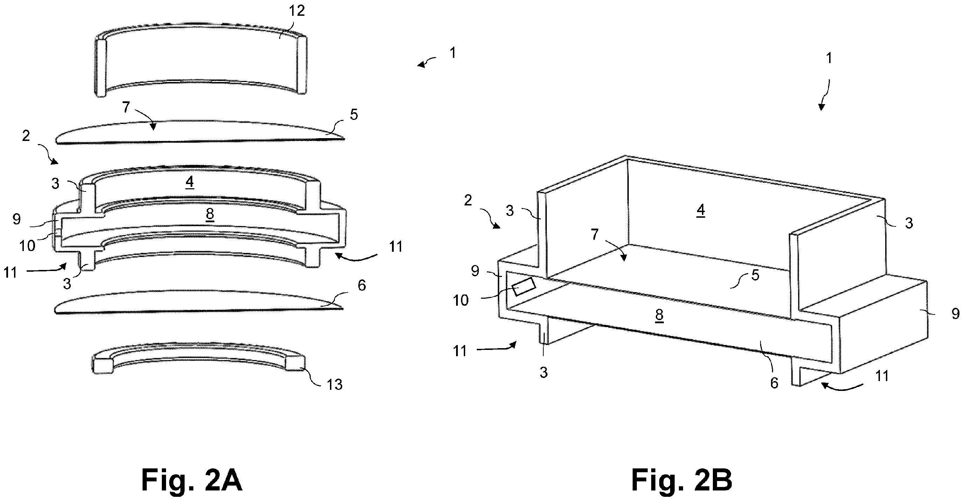

Figure 2A shows an exploded view (in cross section) of the cell stretching device 1 according to an example.

In example, the transversal cross-section of the housing 2 is circular. In particular, the housing 2 has a cylindrical structure comprising a cylindrical air chamber 8 and a cylindrical cell seeding chamber 4. The two chambers are coaxial and the air chamber 8 is larger than the cell seeding chamber 4. In particular, the difference in size between these two chambers is represented by the protruding region 11 as explained above. The circular air chamber 8 can be connected to a vacuum pump through the vacuum port 10 located at one side of the central portion 9 of walls 3 of the housing 2. Since the protruding region 11 extends all around the central portion 9 of the housing 2, this device is suitable for a biaxial stretching.

In examples, the device 1 comprises upper fixing means 12 for fixing the first deformable membrane 5 to the housing 2 and lower fixing means 13 for fixing the second deformable membrane 6 to the housing 2. The fixing means 12, 13 are configured to fix the membranes 5, 6 to the housing 2 in a removable way. In one example, the upper fixing means 12 and the lower fixing means 13 can be circular rings configured to clamp the first membrane 5 and the second membrane 6 to the housing, respectively. For this purpose the diameter of the circular rings can be lower of the diameter of the housing 2. In particular, the upper fixing means 12 is configured to clamp the first deformable membrane 5 to an upper part of the housing 2, for example at the cell seeding chamber 4. The lower fixing means 13 is configured to clamp the second deformable membrane 6 to a lower part of the housing 2, for example opposite to the cell seeding chamber 4. It is noted that the fixing means can be of any nature and can have any shape and dimension provided that they are suitable to fix the membranes 5, 6 to the housing 2, preferably in a removable way. For example, the fixing means can comprise gripping tools, adhesive tools, snap-fit tools, etc.

Figure 2B shows a perspective view of the cell stretching device 1 according to another example. In this example, the transversal cross-section of the housing 2 is not circular but rather rectangular. In particular, the housing 2 has a polyhedric structure comprising a rectangular parallelepiped air chamber 8 and a rectangular parallelepiped cell seeding chamber 4. In addition, differently from the device of Figure 2A, the protruding region 11 does not extend all around the central portion 9 of the housing 2, but extends only along two opposing sides of the central portion 9. Accordingly, the device of figure 2B is suitable for an uniaxial stretching. It is noted that, with the exception of the shape of the housing 2, the device of figure 2B comprises all the technical characteristics of the device of Figure 2A. Therefore, all the characteristics defined in the following with reference to the configuration of the device 1 of figure 2A also apply to the configuration of the device 1 of figure 2B.

In examples, the first deformable membrane 5 and/or the second deformable membrane 6 is fixed to the housing 2 to form a sealing region between the housing 2 and said first and/or second deformable membrane 5, 6. In this way, the air chamber 8 is air sealed from outside. For example, with reference to figure 2, the sealing region is advantageously a circular region at the contact area between the housing 2, i.e. the walls 3, and the fixing means 12, 13.

In examples, the the housing 2 is made of plastic. In particular, the housing 2 can be a one-piece element made of a polymeric material or other material suitable to be printed by means of a 3D-printer. This implies that the device 1 can be mass produced easily by other industrial process and using different materials. The housing can also be made of different materials which are not necessary apt to be printed with a 3D-printer but are suitable for the purpose of the present invention. The fixing means 12, 13 can advantageously be made of the same material of the housing 2.

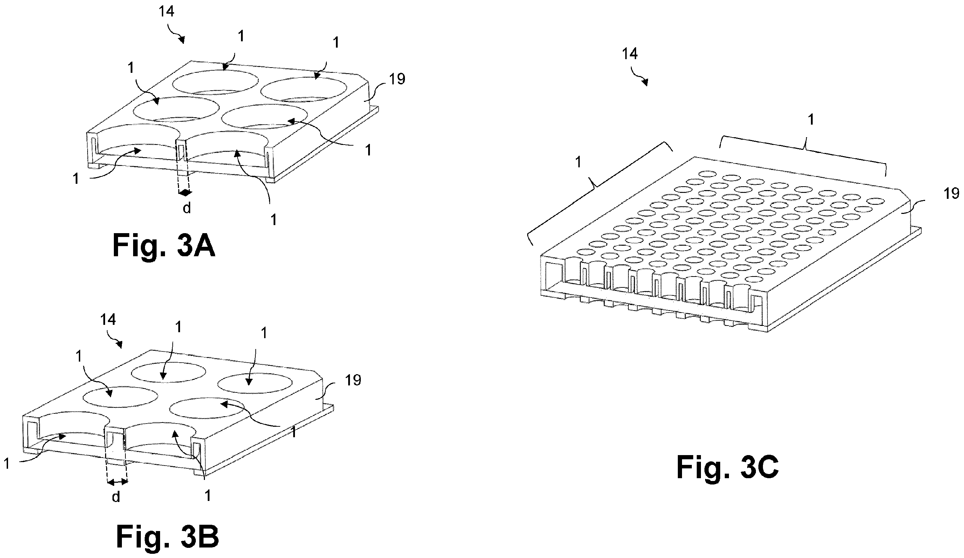

Figures 3A, 3B and 3C show two plates 14 for cell stretching according to two different examples. In particular, the plate 14 of figures 3A and 3B comprises a plurality of cell stretching devices 1 arranged in rows and columns. Specifically, a frame is configured to position six devices 1 in an arrangement 2x3. On the other hand, the plate 14 of figure 3C comprises a frame configured to position ninety-six devices 1 in an arrangement 8x12. The first row of each plate 14 is cut to show that the present cell stretching device 1 and in particular the hollow region/cell seeding chamber 4 can be used as a well for hosting a culture cell substrate, possibly immersed in a growth medium.

The plates 14 of figures 3A and 3B are basically the same. The only difference consists in the distance d between two consecutive devices 1. As shown in the figures, the distance d of the plate 14 of figure 3B is larger than the distance d of the plate 14 of figure 3A. By increasing the value of distance d, the stretching level is increased. These plates 14 represent standard 6-wells and 96-wells plates used in laboratory for cell analysis. It is clear that these are just two examples and that other arrangements are possible, such as for example a 24-wells plate. In an example, the vacuum chamber of the plurality of cell stretching devices 1 of the plate 14 are connected to each other so that a plate vacuum port (not shown in the figure) is present, e.g. at a side of the frame 19 of the plate 14. Accordingly, the plate 14 can be connected to a vacuum system through said plate vacuum port. The connection from each chamber to the plate vacuum port can be carried out by a vacuum duct system inside the frame 19. Advantageously, the chambers are connected to a single plate vacuum port. However, a plurality of plate vacuum ports can be present, each plate vacuum port of said plurality being connected to a row or a column of cell stretching devices 1 (i.e. of vacuum ports 10) as arranged in the plate 14.

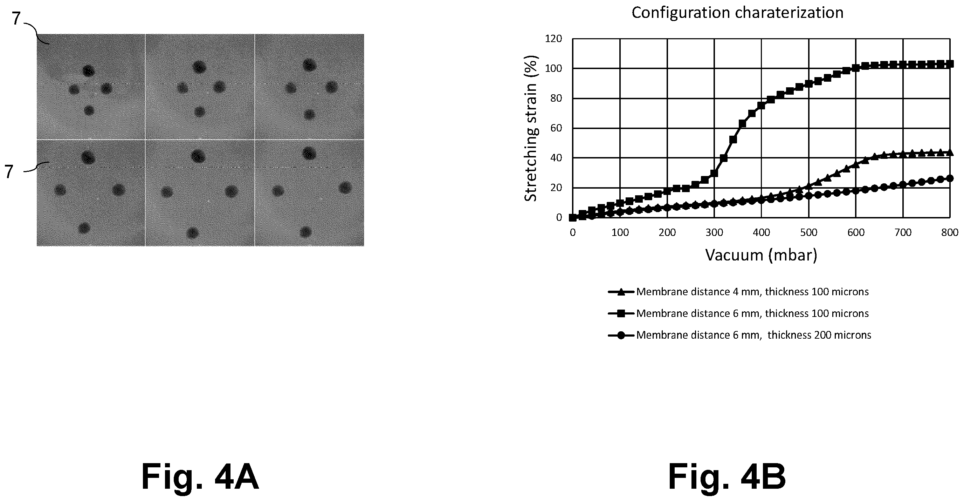

Figures 4A-B illustrate a characterization of configuration of membrane distance d and membrane thickness (membrane size is fixed at 40 mm in diameter) at different vacuum pressure values. Figure 4A shows the cell substrate 7 (marked by four black ink dots) been stretched as vacuum increase from 0 to 800 mbar (membrane distance 6 mm and thickness 100 µm). Figure 4B shows the stretching level (strain) (y-axis) of different configurations versus different vacuum pressure values (x-axis). It is noted from this figure that the stretching level at a fixed vacuum depends on the thickness, size, and distance of the two membranes 5, 6. In one example, with a membrane thickness of 100 µm thickness, a membrane diameter of 40 mm and membrane distance d of 6 mm, the maximum stretching level can increase to 100% after 600 mbar vacuum.

As shown in figure 4B, the maximum stretching level of the cell stretching device is determined by the size of the air chamber 8 and the thickness of the membrane 5, 6. Basically, by increasing the size of the air chamber 8, the maximum stretching level increases. As described above, in one configuration, the stretching level can increase to 100%, meaning that the membrane's surface is doubled by deformation

Accordingly, the configuration of the device 1 can be scaled up to stretch large cell populations for biochemical and genetic analysis. The device 1 can also be miniaturized and used in assays for drug screening.

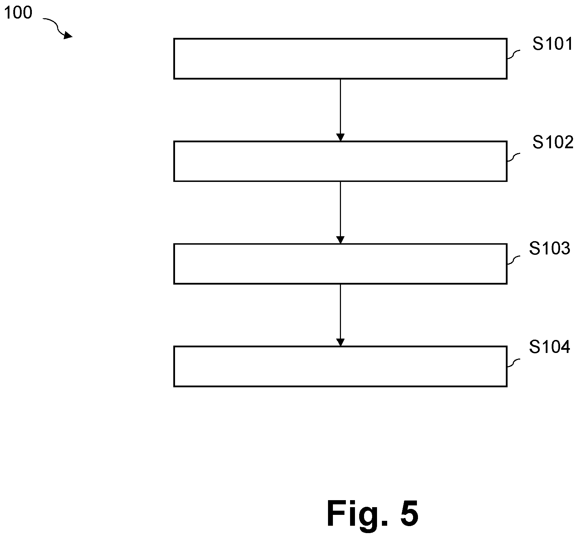

Figure 5 schematically shows the steps of the method 100 for cell stretching using the device 1 or the plate 14 described above.

At step S101, at least one cell stretching device 1 or the plate 14 for cell stretching is provided. Then, one of the first or the second deformable membrane 5, 6 are seeded with cells to be analyzed, thereby forming a deformable cell substrate 7 (at step S102). At step S103, the cells are incubated for a first predetermined period of time and at step S104 the cell substrate 7 is stretched by cyclic aspiration and release of air through the vacuum port 10 of the device 1 or plate 14 for a second period of time.

In examples, the cyclic aspiration and release of air occurs by a vacuum pressure variation inside the air chamber 8 of the device 1 or plate 14, wherein in particular the vacuum pressure varies between 0 mbar and 800 mbar.

The device 1 is designed to enclose two flexible membranes 5, 6 (thickness 0.005 inches, commercialized by SMI Specialty Manufacturing Inc.) and the membrane stretching is achieved by cyclic aspiration and release of air through a negative and a positive air pump. Following device assembly and sterilization (by rinsing it with 70% EtOH), the upper membrane is coated with fibronectin (10 µg/ml), incubated at 37 °C for 1 hour and washed three times with 1× PBS sterile. IMR90 fibroblasts are seeded in the cell chamber 4 (200.000/device) and incubated overnight before starting the stretching. The devices 1 are controlled by a MAT pipeline (Fluigent) programmed to exert a 20% cyclic stretch at 0.2 Hz frequency for a total of 6 hours inside in a humidified atmosphere (5% CO2 , 37°C). Cells seeded on non-stretched devices are used as control. Following 6h of continuous cyclic cell stretching, cells are washed once with 1×PBS, then lysed by adding 150 µl of 2× BOLTTMLDS buffer complemented with 100 mM DTT and scraping the membrane surface with a cell scraper. Lysates are collected in 1.5 ml tubes on ice, sonicated for 10 s at 4 °C then boiled for 10 minutes at 95 °C. Samples are stored at -20 °C until ready to be analysed by western blot.

According to the results, quantifications of H3K9me3 and H3K27me3 relative to total H3 show increased chromatin condensation in cells treated with ATMi and exposed to cyclic stretching.

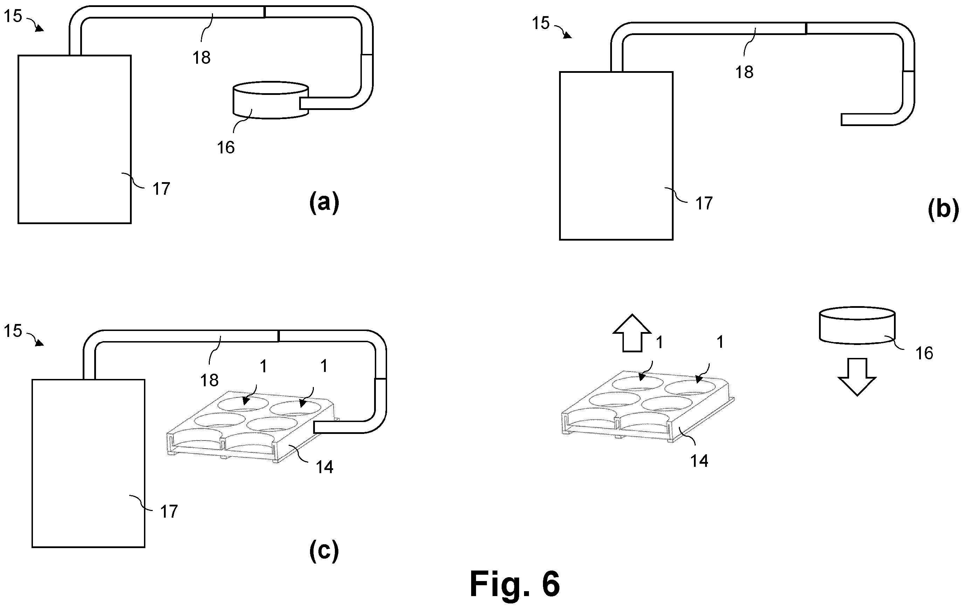

Figure 6 illustrates the steps of a process for retrofitting a standard cell stretching system. In particular, this process is suitable for a cell stretching system that uses a vacuum unit 15 for generating a stretching on a deformable cell substrate. The cell stretching system comprises an apparatus 16 supporting the deformable cell substrate and being configured to be connected to said vacuum unit 15. As shown in figure 6 (a), the vacuum unit 15 comprises at least a pump unit 17 and a piping system 18 connecting the pump unit 17 to the apparatus 16. The process comprises replacing the apparatus 16 with the cell stretching device 1 or the cell stretching plate 14 previously described. The replacement occurs by simply connecting the vacuum unit 15 to the vacuum port 10 of the cell stretching device 1 (figure 6 (b)-(c)). In other words, the performance of a standard cell stretching system can be improved by using the cell stretching device 1 or the cell stretching plate 14 here described, without the necessity of replacing or adding the vacuum unit 15, thereby saving overall costs.

Although a variety of techniques and examples of such techniques have been described herein, these are provided by way of example only and many variations and modifications on such examples will be apparent to the skilled person and fall within the spirit and scope of the present invention, which is defined by the appended claims and their equivalents.

## Claims

1. Cell stretching device (1) comprising:
a housing (2) having walls (3) defining a hollow region (4);
a first deformable membrane (5) and a second deformable membrane (6) facing the first deformable membrane (5), both the first deformable membrane (5) and the second deformable membrane (6) being located in the housing (2) and being fixable to the walls (3) of the housing (2); and
an air chamber (8) formed in an internal region of the housing (2) and defined by the first deformable membrane (5), the second deformable membrane (6), and a central portion (9) of the walls (3) of the housing (2), said central portion (9) of the walls (3) comprising at least a vacuum port (10) for evacuating air from the air chamber (8) and to determine a pressure variation inside the air chamber (8),
wherein in a first configuration the air pressure inside the air chamber (8) is such that the first deformable membrane (5) is separated from the second deformable membrane (6) at a predefined membrane distance (d), and in a second configuration the air pressure inside the air chamber (8) is reduced such that the first deformable membrane (5) and the second deformable membrane (6) are deformed and stretched towards the central portion (9) of the walls (3) of the housing (2) and such that the first deformable membrane (5) is, at least partially, in contact with the second deformable membrane (6) to form a stretching region.

2. Device (1) according to claim 1, wherein the first deformable membrane (5) or the second deformable membrane (6) is a deformable cell substrate (7) for growing cultured cells and a central portion of the hollow region (4) represents a cell seeding chamber.

3. Device (1) according to any one of claims 1 to 2, wherein the air chamber (8) has an average length (I) and an average height (h) and wherein:
a. the height (h) of the air chamber (8) is the predefined membrane distance (d) between the first deformable membrane (5) and the second deformable membrane (6) in the first configuration, and/or
b. the ratio between said height (h) and said length (I) is comprised between 4:40 and 8:40, in particular 6:40; and/or
c. the maximum stretching level of the cell stretching device (1) increases as a function of at least one of said length (I), the membrane distance (d), and the membrane thickness, at a fixed vacuum value inside the air chamber (8).

4. Device (1) according to any one of claims 1 to 3, wherein the thickness of the first deformable membrane (5) and/or of the second deformable membrane (6) is comprised between 0.1 µm and 1000 µm, in particular 100 µm or 200 µm.

5. Device (1) according to any one of claims 1 to 4, wherein the predefined membrane distance (d) in the first configuration is comprised between 0.1 mm and 100 mm, in particular 4 mm or 6 mm.

6. Device (1) according to any one of claims 1 to 5, wherein the first deformable membrane (5) and/or the second deformable membrane (6) is fixed to the housing (2) to form a sealing region between the housing (2) and said first and/or second deformable membrane (5, 6).

7. Device (1) according to any one of claims 1 to 6, further comprising upper fixing means (12) for fixing the first deformable membrane (5) to the housing (2) and lower fixing means (13) for fixing the second deformable membrane (6) to the housing (2).

8. Device (1) according to any one of claims 1 to 7, wherein the housing (2) comprises a protruded region (11) for receiving a portion of the first deformable membrane (5) and of the second deformable membrane (6) when stretched in the second configuration.

9. Device (1) according to any one of claims 1 to 8, wherein
a. the transversal cross-section of the housing (2) is circular for a multiaxial stretching; or
b. the transversal cross-section of the housing (2) is rectangular for an uniaxial stretching; and/or
c. the housing (2) is made of plastic.

10. Device (1) according to any one of claims 1 to 9, wherein the stretching region is a flat region and wherein in the second configuration the first and the second deformable membranes (5, 6) are exclusively in contact with each other and with the walls (3) of the housing (2).

11. Device (1) according to any one of claims 1 to 10, wherein the first deformable membrane (5) and/or the second deformable membrane (6) has a diameter comprised between 1 mm and 100 mm, in particular 40 mm, 2mm, 4mm, 8mm, 16mm, 35mm.

12. Plate (14) for cell stretching comprising:
a plurality of cell stretching devices (1) according to any one of claims 1 to 11, and
at least a frame (19) configured to position each of the plurality of cell stretching devices (1) arranged in rows and columns.

13. Method (100) for cell stretching using the device (1) of any one of claims 1 to 11 or the plate (14) according to claim 12, the method (100) comprising:
- providing (S101) at least one cell stretching device (1) or the plate (14) for cell stretching;
- seeding (S102) one of the first or the second deformable membrane (5, 6) with cells to be analyzed, thereby forming a deformable cell substrate (7);
- incubating (S103) the cells for a first predetermined period of time; and
- stretching (104) the cell substrate (7) by cyclic aspiration and release of air through the vacuum port (10) of the device (1) or plate (14) for a second period of time.

14. Method of claim 13, wherein the cyclic aspiration and release of air occurs by a vacuum pressure variation inside the air chamber (8) of the device (1) or plate (14), wherein in particular the vacuum pressure varies between 0 mbar and 800 mbar.

15. A process for retrofitting a cell stretching system that uses a vacuum unit (15) for generating a stretching on a deformable cell substrate, said cell stretching system comprising an apparatus (16) supporting the deformable cell substrate and being configured to be connected to said vacuum unit (15), the process comprising replacing the apparatus (16) with the cell stretching device (1) according to any one of claims 1 to 11 or the cell stretching plate (14) of claim 12 by connecting the vacuum unit (15) to the vacuum port (10) of the cell stretching device (1).
